## Europäisches Patentamt
## European Patent Office
## Office européen des brevets

(19)

(11) Veröffentlichungsnummer: **0 169 504**
**B1**

(12) # EUROPÄISCHE PATENTSCHRIFT

(45) Veröffentlichungstag der Patentschrift:
**17.05.89**

(21) Anmeldenummer: **85108980.5**

(22) Anmeldetag: **18.07.85**

(51) Int. Cl.⁴: **C12N 15/00**, C12P 19/34,
C12P 21/00, C07K 3/00
// C12R1:19

(54) **Verwendung einer DNA-Sequenz zur Expression sowie DNA-Struktur und Expressionsvektor mit der DNA-Sequenz.**

(30) Priorität: **18.07.84 DE 3426532**

(43) Veröffentlichungstag der Anmeldung:
**29.01.86 Patentblatt 86/5**

(45) Bekanntmachung des Hinweises auf die Patenterteilung:
**17.05.89 Patentblatt 89/20**

(84) Benannte Vertragsstaaten:
**BE DE FR GB IT NL SE**

(56) Entgegenhaltungen:
**Keine**

(73) Patentinhaber: **Gesellschaft für Biotechnologische Forschung mbH (GBF), Mascheroder Weg 1, D-3300 Braunschweig-Stöckheim(DE)**

(72) Erfinder: **Sebald, Walter, Prof. Dr., Am Hopfengarten 42, D-3340 Wolfenbüttel(DE)**
Erfinder: **McCarthy, John Edward Grimwade, Dr., Kurzekampstrasse 3, D-3300 Braunschweig(DE)**

(74) Vertreter: **Boeters, Hans Dietrich, Dr. et al, Boeters, Bauer & Partner Thomas-Wimmer-Ring 14, D-8000 München 22(DE)**

EP 0 169 504 B1

**Beschreibung**

Es ist heute noch weitgehend ungeklärt, welche Eigenschaften der mRNA eine optimale Bindung des Ribosoms und damit eine maximale Translation zum Protein in der Zelle ermöglichen. Die bisherige Diskussion betont die Bedeutung der SD-Sequenz (Shine-Delgano-Sequenz, Ribosomenbindungsstelle), die im richtigen Abstand zum Startcodon liegen muß, sowie die Bedeutung von Sekundärstrukturen der mRNA, die den Start der Translation hemmen. Es gibt aber zahlreiche Hinweise, daß weitere Sequenzelemente der mRNA für eine effektive Bildung des Initiationskomplexes und damit einer optimalen Translation nötig sind. Diese Sequenzelemente sind jedoch noch nicht definiert.

Es ist bekannt, daß das atp-Operon von Escherichia coli 8 Gene enthält, die für die 8 Untereinheiten (a, c, b, delta, alpha, gamma, beta und epsilon) der ATP-Synthase (ATP = Adenosintriphosphat) kodieren. Das atp-Operon wird in eine polycistronische mRNA transscribiert, an der die Untereinheiten in verschiedenen molaren Mengen translatiert bzw. synthetisiert werden. Die höchste Translationsrate wird für die Untereinheit c beobachtet.

Bei der Erfindung zugrundeliegenden Arbeiten wurde das Gen für die Untereinheit c isoliert und in Expressionsvektoren kloniert. Dabei zeigte sich, daß die hohe Translationsrate nur dann beibehalten wird, wenn eine DNA-Sequenz von etwa 40 Basenpaaren vor der Shine-Delgano-Sequenz mitkloniert wird.

Erfindungsgemäß können nun hohe Expressionsraten für Strukturgene in technisch kultivierbaren Bakterien erzielt werden, wenn man die folgende intercistronische DNA-Sequenz des atp-Operons von E. coli für die Untereinheit c der ATP-Synthase

```
T A A                          fakultatives Stopcodon
A T T                          für Untereinheit a

T T T A C C A A C A C T A C T A C G T T   intercistronische
A A A T G G T T G T G A T G A T G C A A   Sequenz atp(a)-atp(c)

T T A A C T G A A A C A A A C T
A A T T G A C T T T G T T T G A

G G A G                        fakultative
C C T C                        Shine-Delgano-Sequenz

A C T G T C A T G                fakultatives Startcodon
T G A C A G T A C                für Untereinheit c
```

(wobei Basentriplets durch ihre Synonyme ersetzt sein können) gegebenenfalls mit unmittelbar vorgeschaltetem Stopcodon für die Untereinheit a der ATP-Synthase, gegebenenfalls mit unmittelbar nachgeschalteter Shine- Delgano-Sequenz und gegebenenfalls mit der Shine-Delgano-Sequenz unmittelbar nachgeschaltetem Startcodon für die Untereinheit c der ATP- Synthase oder

eine etwa 40 Basenpaare umfassende DNA-Sequenz verwendet, deren Einzelstränge mit denen der intercistronischen Sequenz (vorzugsweise bei einer Temperatur von mindestens 20°C und insbesondere bei einer Konzentration von 1 M NaCl und einer Temperatur von mindestens 25°C) hybridisierbar sind.

Zu diesem Zweck wird erfindungsgemäß ferner eine DNA-Struktur mit einer unmittelbar vorgeschalteten endständigen derartigen DNA-Sequenz vorgesehen.

Erfindungsgemäß wird ferner ein Expressionsvektor vorgesehen, der durch eine derartige DNA-Sequenz gekennzeichnet ist.

Beispiel 1

Die intercistronische DNA-Sequenz atp(a)-atp(c) wurde mit DNA für Interleukin-2 rekombiniert, und nach Integration in einen Expressionsvektor und Transformation von E. coli wurde festgestellt, daß die Translation dieses Proteins dramatisch erhöht wurde.

Beispiel 2

Die intercistronische DNA-Sequenz atp(a)-atp(c) wurde mit DNA für β-Interferon rekombiniert, und nach Integration in einen Expressionsvektor und Transformation von E. coli wurde eine erhöhte Ausbeute von β-Interferon erzielt.

**Patentansprüche**

1. Eine erhöhte Proteinexpression bewirkende intercistronische DNA-Sequenz des atp-Operons von E. coli für die Untereinheit c der ATP-Synthase

```
T T T A C C A A C A C T A C T A C G T T
A A A T G G T T G T G A T G A T G C A A


T T A A C T G A A A C A A A C T
A A T T G A C T T T G T T T G A
```

(wobei Basentripletts durch ihre Synonyme ersetzt sein können)
– gegebenenfalls mit unmittelbar vorgeschaltetem Stopcodon für die Untereinheit a der ATP-Synthase,
– gegebenenfalls mit unmittelbar nachgeschalteter Shine-Dalgarno-Sequenz und
– gegebenenfalls mit der Shine-Dalgarno-Sequenz unmittelbar nachgeschaltetem Startcodon für die Untereinheit c der ATP-Synthase
oder
eine etwa 40 Basenpaare umfassende DNA-Sequenz, deren Einzelstränge mit denen der intercistronischen DNA-Sequenz bei einer Temperatur von mindestens 25°C und einer 1 M NaCl-Konzentration hybridisierbar sind, und die gleichfalls eine erhöhte Proteinexpression bewirkt.
2. Expressionsvektor, gekennzeichnet durch eine DNA-Sequenz gemäß Anspruch 1.
3. Verwendung einer DNA-Sequenz gemäß Anspruch 1 zur Expression von Strukturgenen in technisch kultivierbaren Bakterien.
4. Verfahren zur Herstellung eines Proteins, dadurch gekennzeichnet, daß man
(c) ein Bakterium kultiviert, das einen Expressionsvektor gemäß Anspruch 2 aufweist, und das Protein gewinnt, wobei das Verfahren gegebenenfalls eine Stufe
(b) umfaßt, bei der vor Stufe (c) der Expressionsvektor in das Bakterium integriert wird, und wobei das Verfahren gegebenenfalls eine weitere Stufe
(a) umfaßt, bei der vor Stufe (b) eine DNA-Sequenz gemäß Anspruch 1 mit dem Strukturgen des Proteins rekombiniert und die rekombinierte DNA mit einem Vektor rekombiniert und der Expressionsvektor erhalten wird.

**Claims**

1. An intercistronic DNA sequence of the atp operon of E. coli for subunit c of ATP synthase

```
T T T A C C A A C A C T A C T A C G T T
A A A T G G T T G T G A T G A T G C A A


T T A A C T G A A A C A A A C T
A A T T G A C T T T G T T T G A
```

(wherein base triplets may be replaced by their synonyms)
– optionally with a stop codon for subunit a of ATP synthase immediately upstream,
– optionally with a Shine-Dalgarno sequence immediately downstream and
– optionally with a start codon for subunit c of ATP synthase immediately downstream of the Shine-Dalgarno sequence,
which DNA sequence brings about increased protein expression
or
a DNA sequence that comprises approximately 40 base pairs and of which the single strands can be hybridised with those of the intercistronic DNA sequence at a temperature of at least 25°C and at a 1 M concentration of NaCl and that likewise brings about increased protein expression.
2. An expression vector, characterised by a DNA sequence according to claim 1.
3. The use of a DNA sequence according to claim 1 for the expression of structural genes in industrially cultivatable bacteria.

4. A process for the preparation of a protein, characterised in that

(c) a bacterium containing an expression vector according to claim 2 is cultivated, and the protein is recovered, the process optionally comprising a step (b) in which, before step (c), the expression vector is integrated into the bacterium, and the process optionally comprising a further step

(a) in which, before step (b), a DNA sequence according to claim 1 is recombined with the structural gene of the protein and the recombined DNA is recombined with a vector and the expression vector is obtained.

**Revendications**

1. Séquence d'ADN intercistronique de l'atp-opéron d'E. Coli pour la sous-unité c de l'ATP-synthase séquence qui provoque une expression élevée en protéine et qui est caractérisée en ce qu'elle a la composition suivante:

T T T A C C A A C A C T A C T A C G T T

A A A T G G T T G T G A T G A T G C A A


T T A A C T G A A A C A A A C T

A A T T G A C T T T G T T T G A

(dans cette formule, des triplets de bases peuvent être remplacés par leurs synonymes)

– éventuellement avec, placé juste avant, un codon d'arrêt pour la sous-unité a de l'ATP-synthase,

– éventuellement avec, placée just après, une séquence de Shine-Dalgarno, et

– éventuellement avec un codon de départ pour la sous-unité c de l'ATP-synthase, placé juste après la séquence de Shine-Dalgarno, ou

séquence d'ADN qui renferme environ 40 paires de bases et dont les brins élémentaires peuvent être hybridés avec ceux de la séquence d'ADN intercistronique à une température d'au moins 25°C et à une concentration 1 M de NaCl, et qui provoque également une expression élevée en protéine.

2. Vecteur d'expression caractérisé par une séquence d'ADN selon la revendication 1.

3. Application d'une séquence d'ADN selon la revendication 1 pour l'expression de gènes de structure dans des bactéries cultivables industriellement.

4. Procédé pour préparer une protéine, procédé caractérisé en ce que:

(c) on cultive une bactérie qui présente un vecteur d'expression selon la revendication 2, et on isole la protéine, ce procédé comprenant éventuellement:

(b) une étape dans laquelle, avant l'étape (c), on intègre le vecteur d'expression dans la bactérie, et éventuellement,

(a) une étape supplémentaire dans laquelle, avant l'étape (b), on recombine une séquence d'ADN selon la revendication 1 avec le gène de structure de la protéine et on recombine avec un vecteur l'ADN recombiné, opération qui donne le vecteur d'expression.